# EUROPEAN PATENT SPECIFICATION

(11) **EP 1 961 437 B1**
(45) Date of publication and mention of the grant of the patent: **26.10.2011**
(21) Application number: 08425090.1
(22) Date of filing: 14.02.2008
(51) Int. Cl.: A61M 5/28, A61M 5/32

(54) **Instrument for injections with retractable needle following use**
Vorrichtung für Injektionen mit nach der Anwendung einziehbarer Nadel
Instruments pour injections dont l'aiguille peut se rétracter après utilisation

(30) Priority: 26.02.2007 IT BS20070024
(43) Date of publication of application: 27.08.2008
(73) Proprietor: Boldini, Fabio, 25080 Puegnago del Garda (Brescia) (IT)
(72) Inventor: Boldini, Fabio, 25080 Puegnago del Garda (Brescia) (IT)
(74) Representative: Sangiacomo, Fulvia

(56) References cited:
- EP-A- 1 410 818
- WO-A-92/00701
- WO-A-93/03781
- GB-A- 1 217 152

## Description

### Field of the Invention

This invention concerns the medical field and refers in particular to an instrument for intramuscular and subcutaneous injections of the type comprised of a ampoule or phial of medicinal substances connected to a needle for injections.

### State of the Art

In the field taken into consideration, an instrument for injections that comprises a container shaped as an ampoule or phial, of a flexible material, preloaded or loadable, and a needle for injections supported by a connecting element is already known. The medicinal container has a neck and the needle is coupled to said neck either by being screwed or pressed into it.

Initially, the neck of the container is sealed, but it may be perforated; the needle has a rear perforating end facing towards the neck; the connecting element is constrained in a first position relatively to the neck of the container so that the rear perforating end of the needle is at a distance from the neck so as not to perforate it; the needle is enclosed in a protective cap. To inject the medicinal substance, the connecting element is tightly engaged on the neck of the container in a second position so that the perforating end of the needle will perforate the neck and thus be in communication with the inside of the container. Then, once the protective cap has been removed, the injection can be executed by compressing the container obliging the medicinal substance to exit from the needle towards the patient.

However, after the injection and when it has been extracted from the patient, the needle remains in a fixed position and exposed with the risk of injuring/contaminating anybody accidentally coming into contact with it, even only when replacing the protective cap.

Technically and commercially, syringes with a retractable needle following use so as to avoid the risk of contamination are well known, but as far as we know the retractable technique of the needle has never been applied to instruments of the type described above.

In particular, the patent application WO 93/03781 relates to a unit dose syringe comprises a fluid-containing plastic bag with an attached needle and a dimensionally-stable shell like a woman's compact. The shell contains a thumb-operated slider assembly for advancing the needle to an exposed position for fluid delivery. The syringe also includes a spring-biasing arrangement to return the needle, automatically, to the protected position, after use, and a means for rupturing the bag and retaining the needle in the protected position.

The patent application EP 1410818 relates to a vial for injection comprising: an elastically deformable container wherein a solution for injection is hermetically contained, and a male connector protruding forward from said container to engage in a female connector of a needle-carrier which bears a needle. The needle has a first tip which protrudes forward from the needle-carrier to engage in the patient's body and a second tip disposed in the female connector of the needle-carrier to perforate the end of said male connector of the vial so that, by means of manual pressure on the container of the vial, the solution contained therein can be injected through the needle into the patient's body.

The patent application GB 1217152 relates to a syringe, eye-dropper or the like, in which a cannula is forced into a sealed flexible container to deliver or collect a liquid therein, comprises (a flexible container having a plug at one end carrying an external screw thread, on to which fits the internal screw thread of the member carrying the needle. A cover fits over the needle and carrying member and abuts against a shoulder at the base of the container. The cover is a friction fit on a part of the member having longitudinal serrations, but both the cover and the members are slightly tapered and/or the cover includes a wider section so that, when the cover is rotated, the needle-carrying member moves inwardly along the screw thread towards the container until the needle pierces the plug, by which time the cover and member have disengaged and the cover can be removed.
With the devices cited in these cited patent applications, an user can accidently move the needle, after its use.

### Object and Summary of the Invention

The object of this invention is to remedy this shortcoming and, therefore, create the conditions to protect against any contact and risk of contamination those instruments for injections that use a compressible, preloaded or loadable container with a medicinal substance, connected to a needle for injections moving between a first neutral position and a second perforating position of the container.

Such an object is reached, according to the invention, with a container of medicinal substance according to the preface of claim 1, wherein the needle is fixed using a needle-holder block provided in the connection element and moving between a forward position in which the needle extends forward beyond the connecting element and a retracted position in which the needle is fully retracted inside the connecting element, and wherein the forward position of the needle-holder block is set by a catch moving between an engaging and a release position of said needle-holder block with said block and the retracted position is originated by a return spring in response to the release of said needle-holder block by the catch.

The container can be produced both complete with medicinal substance, or empty, to be loaded later by the end operator: by compressing the body of the container to make the air it contains exit and by taking advantage of the consequent return to fill it with medicinal substance.

### Brief Description of the Drawings

The invention will however be illustrated in greater detail in the description to follow in reference to the attached indicative and not limiting drawings, in which;

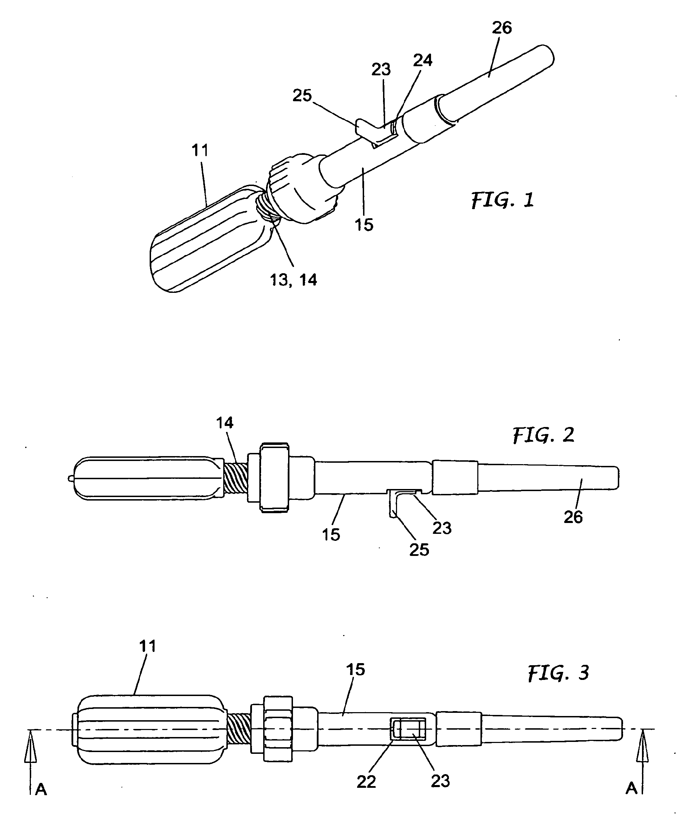

Fig. 1 shows a view in perspective of the instrument for injections with needle protected by a cap;
Figs. 2 and 3 show two exterior views of the instrument in Fig. 1 in right-angled directions;

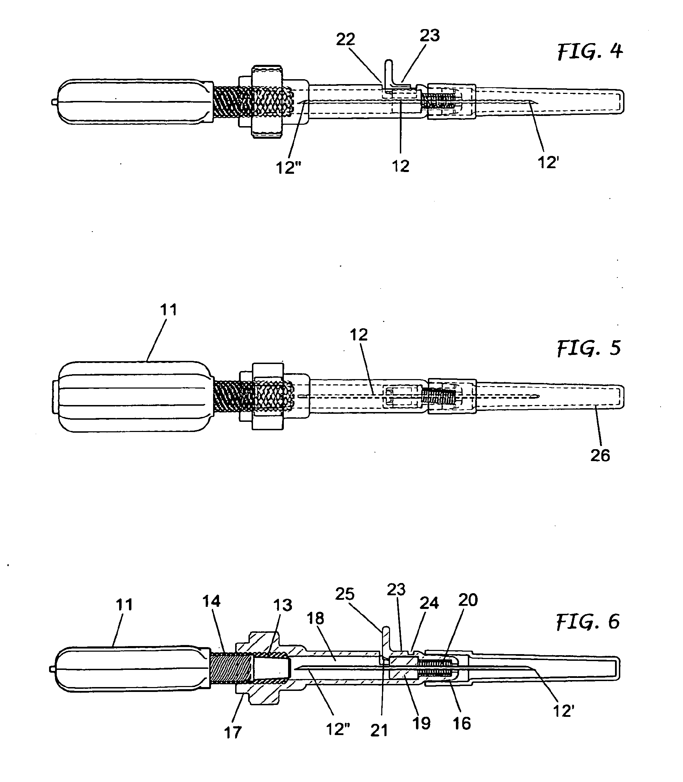

Figs. 4 and 5 show two similar external views of the instrument with internal parts sketched in dotted lines to indicate the arrangement:;
Fig. 6 shows a longitudinal section according to arrows A-A in Fig. 2; and
Fig. 7 shows a section plane of the instrument with needle retracted.

### Detailed Description of the Invention

As represented, the instrument for injections comprises a container 11 made of a flexible material in the shape of a compressible ampoule or phial and a needle for injections 12 associated with it. The container 11 is filled with an injectable medicinal substance and has a neck 13 that is sealed and has a threaded external section 14. The needle 12 is attached to the neck 13 of the container by means of a connecting device 15 with a perforated distal part 16 and a proximal part 17 that is threaded and connects by screwing into the threaded section 14 of said neck. The needle 12 has a front end 12' facing towards the distal end 16 of the connecting element and a rear end 12" facing towards the neck 13 of the container 11.

More precisely, the connecting element 15 internally forms an axial housing 18, and the needle 12 is fixed to a needle-holder block 19, that is housed inside with the possibility of sliding in the housing 18 of said connecting element 15. The needle-holder block 19, including the needle 12, are able to move in said housing 18 between a forward position towards the distal end 16 and a retracted position towards the proximal part 17 of the connecting element 15.

In the example illustrated, after, between the needle-holder block 19 and the distal end 16 of the connecting element 15 there is a compressible return spring 20 provided.

Worthy of note in this case is that the needle-holder block 19 and the return spring can be both simply associated individual components, and elements preventively constrained to each other, for example by co-moulding of the needle-holder block 19, needle 12 and spring 20.

In any case, when the needle-holder block 19 is in the forward position the needle 12 protrudes from the perforated distal end 16 of the connecting element 15. In this position, the return spring 20 is compressed between the needle-holder block 19 and the distal part 16 of the connecting element, and the needle-holder block 19 is held in position by means of a catch 21, positioned at level with the opening 22 in one side of the connecting element 15 and movable between an active blocking position and an inactive release position of the needle-holder block.

The catch 21 is part of a lever 23 that can be integrally machined with the connecting element 15 and connected with the latter by means of an oscillation fulcrum 24. Furthermore, the catch 21 can be devised so that when it is in the active blocking position it engages with the rear of the needle-holder block 19 as shown in the drawings, or alternatively with an intermediate part of said recess, not shown.

The lever 23 normally remains in a state in which the catch 21 is in the active blocking position of the needle-holder block 19, but can be manually moved out of said position by means of a grip 25 it is an integral part of, so as to move the catch 21 away from the needle-holder block 19 and thus release the latter so it can move backward towards the proximal end 17 of the connecting element 15.

Initially, the container 11 is filled with a medicinal substance and its neck 13 is sealed. The needle-holder block 19 is held in its forward position with the needle protruding from the distal part 16 of the connecting element 15. This connecting element 15 is screwed to the container, but only partially until the rear end 12" of the needle reaches the neck 13. The portion of the needle 12 that protrudes from the connecting element 15 is subsequently enclosed in a protective cap 26 - Figs-4-6.

When administering the medicinal substance to a patient the connecting element 15 is screwed tightly onto the neck 13 of the container 11 so that the rear end 12" of the needle 12 will perforate the neck. In this way the needle is now in communication with the inside of the container 11 and after removing the protective cap 26, front end 12' of the needle can be inserted into the body of the patient to inject the medicinal substance.

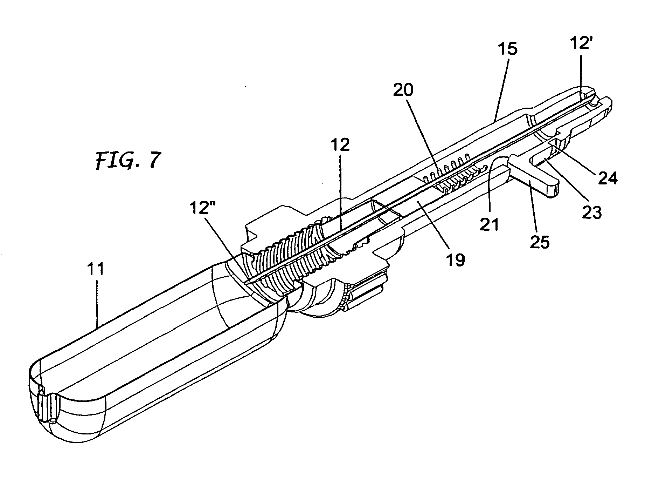

Once the injection has been carried out and the needle extracted from the body of the patient, the operator can use the lever 23 to move the catch 21 away from the needle-holder block 19 and release the latter. Consequently, driven by the return spring 20, the needle-holder block 19 moves back towards the neck 13 of the container until the needle is completely inside the connecting element -Fig. 7- and therefore in a protected condition without the risk of contact with its front end.

It should also be understood that small changes to details can be added to the group described above without however moving outside the sphere of the invention and that the system whereby the needle moves back inside the connecting element may be suitable and applied also to normal syringes and to every other device that comprises a needle for infusions or injections where it would be ideal if it could move back and be concealed after use.

## Claims

1. An instrument for injections with a retractable needle after use, comprising a container for a medicinal substance in the shape of an ampoule or phial made of a flexible material, and a needle for injections supported by a connecting element, where:
- said container has a sealed neck with external threading and the needle is connected to it by screwing onto said neck by means of the connecting element;
- the needle has a front end designed to protrude forwards from the connecting element and a rear end facing towards the neck of said container;
- initially the connecting element is only partially screwed to the neck so that the rear end of the needle is distant from said neck; and said connecting element is tightly screwed to the neck of the container so that the rear end of the needle perforates it at the moment the instrument is used for an injection,
the needle (12) is fixed to a needle-holder block (19) provided in said connecting element (15) and moving between a forward position in which the needle extends forwards beyond the connecting element and a back position in which the needle is completely withdrawn inside the connecting element, the forward position of the needle-holder block being established by a catch (21) moving between an interception position and a release position of said the needle-holder block (19), while the retracted position of said needle-holder block is provoked by the return spring (20) in answer to the release of the needle-holder block on the part of the catch (21),
***characterised in that** said connecting element (15) has* a *perforated distal end (16) and a threaded proximal end that connects to the neck of the container, in which the needle-holder block (19) is positioned and slides in said housing (18) between said distal and proximal end, and **in that** the return spring (20) is placed and loaded between the needle-holder block and the distal end of the connecting element when the block is held in the forward position.*

2. Instrument for injections according to claims 1, in which the catch (21) of the needle-holder block (19) is part of a lever (23) connected to the connecting element (15) by means of an oscillating fulcrum (24) and provided with a gripper tang (25) so as to shift the catch from the interception position to the release position of the needle-holder block.

3. Instrument for injections according to claim 2, in which the catch (21) is positioned level with an opening (22) in one side of the connecting element (15) engaging with the rear of the needle-holder block (19) to hold the latter in the forward position.

4. Instrument for injections according to claim 2 3, in which the catch (21) is positioned level with an opening (22) in one side of the connecting element (15) engaging with an intermediate part of the needle-holder block (19)

5. Instrument for injections according to any of the previous claims, in which the needle-holder block (19) and the return spring (20) are individually associated elements.

6. Instrument for injections according to any one of claims 1-4, in which the needle-holder block (19) and the return spring (20) are preassembled by co-moulding of said block on the end of said spring.

7. Safety system to prevent the re-use of a needle for injections so as to avoid injury and/or infection of anybody who comes into contact with it after it has been used, applicable to ampoules or syringes and including a connecting element complete with needle, needle-holder block, return spring, blocking/releasing needle system and protective cap according to any of the previous claims.

## Patentansprüche

1. Vorrichtung für Injektionen mit nach der Anwendung einziehbarer Nadel, umfassend einen ampullenförmigen Medikamentbehälter aus einem flexiblen Material und eine Nadel für Injektionen, die von einem Verbindungselement gehalten wird, wobei:
- der Behälter einen versiegelten Hals mit Außengewinde hat und die Nadel zum Einschrauben in den Hals mit dem Verbindungselement gekoppelt ist;
- die Nadel ein vorderes Ende besitzt, das dazu bestimmt ist, nach vorn aus dem Verbindungselement überzustehen und ein hinteres Ende, das zum Hals des Behälters gerichtet ist;
- das Verbindungselement ursprünglich nur teilweise in den Hals eingeschraubt ist, damit das hintere Ende der Nadel vom Hals entfernt ist; und wobei das Verbindungselement bis zum Anschlag in den Hals des Behälters eingeschraubt ist, damit das hintere Ende der Nadel diesen bei Verwendung der Vorrichtung für eine Injektion durchstößt;
- die Nadel (12) an einem Nadelhalterblock (19) befestigt ist, der in dem Verbindungselement (15) angeordnet und beweglich ist zwischen einer vorgeschobenen Position, in der die Nadel sich über das Verbindungselement hinaus nach vorn erstreckt, und einer eingezogenen Position, in der die Nadel vollkommen ins Innere des Verbindungselements eingezogen ist, wobei die vorgeschobene Position des Nadelhalterblocks durch einen Arretierzahn (21) bestimmt wird, der zwischen einer Absperrposition und einer Freigabeposition des Nadelhalterblocks (19) beweglich ist, während die eingezogene Position des Nadelhalterblocks durch eine Rücklauffeder (20) als Reaktion auf die Freigabe des Nadelhalterblocks durch den Arretierzahn (21) bewirkt wird,
**dadurch gekennzeichnet, dass** das Verbindungselement (15) ein gelochtes, distales Ende (16) und ein proximales Gewindeende besitzt, das mit dem Hals des Behälters verbunden wird, in dem der Nadelhalterblock (19) angeordnet ist und in diesem Sitz (18) zwischen dem distalen und dem proximalen Ende gleitet, sowie dadurch, dass die Rücklauffeder (20) zwischen dem Nadelhalterblock und dem distalen Ende des Verbindungselements angeordnet ist und gespannt wird, wenn der Block in der vorgeschobenen Position gehalten wird.

2. Vorrichtung für Injektionen nach Anspruch 1, wobei der Arretierzahn (21) des Nadelhalterblocks (19) Teil eines Hebels (23) ist, der mit dem Verbindungselement (15) durch einen Drehpunkt (24) verbunden ist und eine Greifrippe (25) besitzt, um den Arretierzahn von der Absperrposition in die Freigabeposition des Nadelhalterblocks zu verschieben.

3. Vorrichtung für Injektionen nach Anspruch 3, wobei sich der Arretierzahn (21) auf der Ebene einer Öffnung (22) auf einer Seite des Verbindungselements (15) befindet und in die Rückseite des Nadelhalterblocks (19) eingreift, um diesen in der vorgeschobenen Position zu halten.

4. Vorrichtung für Injektionen nach Anspruch 2, wobei der Arretierzahn (21) auf der Ebene einer Öffnung (22) auf einer Seite des Verbindungselements (15) befindet und in einen Zwischenteil des Nadelhalterblocks (19) eingreift.

5. Vorrichtung für Injektionen nach einem beliebigen der vorstehenden Ansprüche, wobei der Nadelhalterblock (19) und die Rücklauffeder (20) einzelne, miteinander verbundene Elemente sind.

6. Vorrichtung für Injektionen nach einem beliebigen der Ansprüche 1 bis 4, wobei der Nadelhalterblock (19) und die Rücklauffeder (20) durch Überpressen des Blocks auf ein Ende der Feder vormontiert sind.

7. Sicherheitssystem zur Verhinderung einer Wiederverwendung der Nadel für Injektionen, um jede Verletzungs- und/oder Kontaminationsgefahr durch Kontakt nach Gebrauch derselben zu vermeiden, welches bei Ampullen oder Spritzen angewendet werden kann und ein Verbindungselement, komplett mit Nadel, Nadelhalterblock, Rücklauffeder, Nadelsperr-/- entsperrsystem und Schutzkappe nach einem beliebigen der vorstehenden Ansprüche umfasst.

## Revendications

1. Instrument pour injections avec une aiguille rétractable après utilisation, comprenant un récipient pour un médicament sous forme d'ampoule ou de fiole d'un matériau flexible, et une aiguille pour injections portée par un élément de raccord, où :
- ledit récipient a un col scellé avec filetage externe et l'aiguille est couplée par vissage audit col à travers l'élément de raccord ;
- l'aiguille a une extrémité antérieure destinée à dépasser vers l'avant de l'élément de raccord et une extrémité postérieure vers le col dudit récipient;
- à l'origine, l'élément de raccord n'est que partiellement vissé au col de sorte que l'extrémité postérieure de l'aiguille soit distante dudit col ; et ledit élément de raccord est vissé à fond sur le col du récipient de sorte que l'extrémité postérieure de l'aiguille le perfore au moment d'utiliser l'instrument pour une injection,
- l'aiguille (12) est fixée à un bloc porte-aiguille (19) disposé dans ledit élément de raccord (15) et mobile entre une position avancée dans laquelle l'aiguille s'étire vers l'avant au-delà de l'élément de raccord et une position rétractée dans laquelle l'aiguille est complètement retirée à l'intérieur du raccord, la position avancée du bloc porte-aiguille étant définie par un cran (21) mobile entre une position d'interception et une position de libération dudit bloc porte-aiguille (19), tandis que la position rétractée dudit bloc porte-aiguille est causée par un ressort de rappel (20) en réponse à la libération du bloc porte-aiguille par le cran (21),
**caractérisé en ce que** ledit élément de raccord (15) a une extrémité distale percée (16) et une extrémité proximale filetée qui s'unit au col du contenant, dans lequel le bloc porte-aiguille (19) est disposé et coulisse dans ledit siège (18) entre lesdites extrémités proximale et distale, et **en ce que** le ressort de rappel (20) est disposé et chargé entre le bloc porte-aiguille et l'extrémité distale de l'élément de raccord lorsque le bloc est retenu dans la position avancée.

2. Instrument pour injections selon la revendication 1, dans lequel le cran (21) du bloc porte-aiguille (19) fait partie d'un levier (23) relié à l'élément de raccord (15) à travers un centre d'oscillation (24) et ayant une ailette de prise (25) de manière à déplacer le cran de la position d'interception à la position de libération du bloc porte-aiguille.

3. Instrument pour injections selon la revendication 3, dans lequel le cran (21) est placé au niveau d'une ouverture (22) dans un côté de l'élément de raccord (15) et s'engage avec l'arrière du bloc porte-aiguille (19) pour retenir ce dernier dans la position avancée.

4. Instrument pour injections selon la revendication 2, dans lequel le cran (21) est placé au niveau d'une ouverture (22) dans un côté de l'élément de raccord (15) et s'engage avec une partie intermédiaire du bloc porte-aiguille (19).

5. Instrument pour injections selon une quelconque des revendications précédentes, dans lequel le bloc porte-aiguille (19) et le ressort de rappel (20) sont des éléments individuels associés.

6. Instrument pour injections selon une quelconque des revendications 1-4, dans lequel le bloc porte-aiguille (19) et le ressort de rappel (20) sont pré-assemblés par surmoulage dudit bloc sur une extrémité dudit ressort.

7. Système de sécurité pour empêcher la réutilisation d'une aiguille pour injections afin d'éviter les blessures et/ou la contagion de quiconque qui entre en contact avec celle-ci après utilisation, applicable à des ampoules ou des seringues et incluant un élément de raccord avec aiguille, bloc porte-aiguille, ressort de rappel, système de verrouillage/déverrouillage d'aiguille et capuchon de protection selon une quelconque des revendications précédentes.
